# EUROPEAN PATENT SPECIFICATION

(11) **EP 3 365 013 B1**
(45) Date of publication and mention of the grant of the patent: **09.12.2020**
(21) Application number: 16858309.4
(22) Date of filing: 21.10.2016
(51) Int. Cl.: A61K 39/395, A61K 9/107, A61K 45/06, C12N 15/113

(54) **METHODS AND RELATED COMPOSITIONS FOR THE TREATMENT OF CANCER**
VERFAHREN UND VERWANDTE ZUSAMMENSETZUNGEN ZUR BEHANDLUNG VON KREBS
MÉTHODES ET COMPOSITIONS ASSOCIÉES POUR LE TRAITEMENT DU CANCER

(30) Priority: 23.10.2015 US 201562245813 P
(43) Date of publication of application: 29.08.2018
(73) Proprietor: Immix Biopharma, Inc., Los Angeles, California 90064 (US)
(72) Inventor: RACHMAN, Ilya, Los Angeles, California 90064 (US)
(74) Representative: Hutter, Anton
(86) International application number: PCT/US2016/058159
(87) International publication number: WO 2017/070494

(56) References cited:
- US-A1- 2010 239 652
- US-A1- 2011 268 655
- US-A1- 2012 178 139
- US-A1- 2015 110 877
- ABOUZEID ABRAHAM H ET AL: "Anti-cancer activity of anti-GLUT1 antibody-targeted polymeric micelles co-loaded with curcumin and doxorubicin", JOURNAL OF DRUG TARGE,, vol. 21, no. 10, 1 December 2013 (2013-12-01), pages 994-1000, XP008177503, ISSN: 1029-2330, DOI: 10.3109/1061186X.2013.840639 [retrieved on 2013-10-07]
- AHMAD FAWZI HUSSAIN ET AL: "Targeted Delivery of Dendritic Polyglycerol-Doxorubicin Conjugates by scFv-SNAP Fusion Protein Suppresses EGFR + Cancer Cell Growth", BIOMACROMOLECULES, vol. 14, no. 8, 19 June 2013 (2013-06-19), pages 2510-2520, XP55248939, US ISSN: 1525-7797, DOI: 10.1021/bm400410e
- NELLIS D F ET AL: "Preclinical manufacture of anti-her2 liposome-inserting, scFv-PEG-lipid conjugate. 2. Conjugate micelle identity, purity, stability, and potency analysis", BIOTECHNOLOGY PROGRESS, AMERICAN CHEMICAL SOCIETY, vol. 21, no. 1, 1 January 2005 (2005-01-01), pages 221-232, XP002467025, ISSN: 8756-7938, DOI: 10.1021/BP049839Z

## Description

### RELATED APPLICATION

This application claims the benefit of priority of U.S. Provisional Patent Application No. 62/245,813, filed on October 23, 2015.

### FIELD OF THE INVENTION

The present disclosure relates to the field of medicine, and more specifically, the treatment of cancer.

### BACKGROUND OF THE DISCLOSURE

Complete and effective treatment for cancer has not been developed despite billions of dollars being spent in cancer research. Part of the reason is because tumor cells can be made up of a variety of cell types, produced as the cells proliferate and incur different mutations. This diversity, in turn, is part of what has made treatment of cancer so difficult, as a population of cancerous cells could easily include a mutant variety that happens to be resistant to any individual treatment or chemotherapy drug that is administered. The few resistant cancer cells are provided a strong selective advantage in comparison to other cells, and over time, those resistant cells increase in frequency.

Abouzeid et al "Anti-cancer activity of anti-GLUT1 antibody-targeted polymeric micelles co-loaded with curcumin and doxorubicin", Journal of Drug Targ., vol. 21, no. 10, 2013, pages 994-100 and US2015/110877 disclose micelles constructs with curcumin and doxorubicin and an anti-GLUT1 antibody against cancer.

Thus, there is a need in the art for the development of additional cancer treatments, including those that have the ability to better target drug resistant tumors and potentially bypass the diversity of cancer cells.

### SUMMARY OF THE INVENTION

The invention is as defined in the claims.

In one aspect, there is provided a composition comprising:
a. a micelle construct;
b. an antibody single chain fragment variable (scFv);
c. a NF-kb inhibitor, which is curcumin and/or salt thereof; and
d. a topoisomerase II inhibitor, which is dox and/or salt thereof.

In another aspect, there is provided a nanoconjugate, comprising:
a. a micelle construct;
b. an antibody single chain fragment variable (scFv);
c. an NF-kb inhibitor, which is curcumin and/or salt thereof; and
d. a topoisomerase II inhibitor which is dox and/or salt thereof;
wherein the micelle construct, the antibody single chain fragment variable (scFv), the NF-kb inhibitor, and the topoisomerase II inhibitor are conjugated to one another.

In one embodiment, disclosed herein are compositions, comprising, a micelle construct; an antibody single chain fragment variable (scFv); a NF-kb inhibitor; and a topoisomerase II inhibitor. In one embodiment, the scFv is conjugated to the micelle construct. In one embodiment, the micelle construct is conjugated to the NF-kb inhibitor. In one embodiment, the micelle construct is conjugated to the NF-kb inhibitor and the topoisomerase II inhibitor. In one embodiment, the NF-kb inhibitor is curcumin and/or salt thereof. In one embodiment, the topoisomerase II inhibitor is dox and/or salt thereof. In one embodiment, the scFv is a glut-1_1 or glut-1_2 scFv. In one embodiment, the micelle construct is targeted to bind to glut-1_1 and/or glut-1_2 scFv. In one embodiment, the composition forms a targeted micelle. In one embodiment, the molecular size of the targeted micelle is 30 nm or less. In one embodiment, the composition further comprises a pharmaceutically acceptable carrier.

In an example, disclosed herein are methods of delivering a drug molecule to a tumor site of a subject comprising: attaching the drug molecule to a targeted micelle, wherein the targeted micelle comprises a micelle construct and an antibody single chain fragment variable (scFv); and delivering the drug molecule attached to the targeted micelle to the tumor site through intracellular delivery. In one embodiment, the drug molecule comprises dox and/or curcumin and/or salt thereof. In one embodiment, the molecular size of the micelle is 30 nm or less. In one embodiment, the tumor is a doxorubicin-resistant tumor. In one embodiment, the subject is human.

In another example, disclosed herein are methods of treating cancer comprising administering to a subject in need thereof, a therapeutically effective dosage of a composition comprising a micelle construct, an antibody single chain fragment variable (scFv), a NF-kb inhibitor, and a topoisomerase II inhibitor. In one example, the composition is administered to the subject intravenously. In one embodiment, the subject is human. In one embodiment, the cancer is brain cancer. In one embodiment, the cancer is ovarian cancer. In one embodiment, the cancer is colon cancer. In one embodiment, the cancer is a doxorubicin-resistant cancer. In one embodiment, the molecular size of the targeted micelle is 30 nm or less.

In another example, disclosed herein are methods of inhibiting cell growth of a tumor cell, comprising: providing a composition comprising a micelle construct, an antibody single chain fragment variable (scFv), a NF-kb inhibitor and a topoisomerase II inhibitor; and inhibiting cell growth by administering a therapeutically effective dosage of the composition to the tumor cell. In one embodiment, the topoisomerase inhibitor comprises Dox. In one embodiment, the NF-kB inhibitor comprises curcumin. In one embodiment, the tumor cell is a brain cancer cell, a ovarian cancer cell, and/or colon cancer cell.

In another embodiment, disclosed herein are nanoconjugates comprising: a micelle construct; an antibody single chain fragment variable (scFv); a NF-kb inhibitor; and a topoisomerase II inhibitor; and wherein the micelle construct, the antibody single chain fragment variable (scFv), the NF-kb inhibitor, and the topoisomerase II inhibitor are conjugated to one another. In one embodiment, the nanoconjugate has a molecular size between 20 nm and 50 nm. In one embodiment, the nanoconjugate has a molecular size between 10 nm and 20 nm. In one embodiment, the nanoconjugate has a molecular size of less than 20 nm. In one embodiment, the nanoconjugate is enclosed by a micelle.

### DESCRIPTION OF THE DRAWINGS

Exemplary embodiments are illustrated in referenced figures. It is intended that the embodiments and figures disclosed herein are to be considered illustrative rather than restrictive.

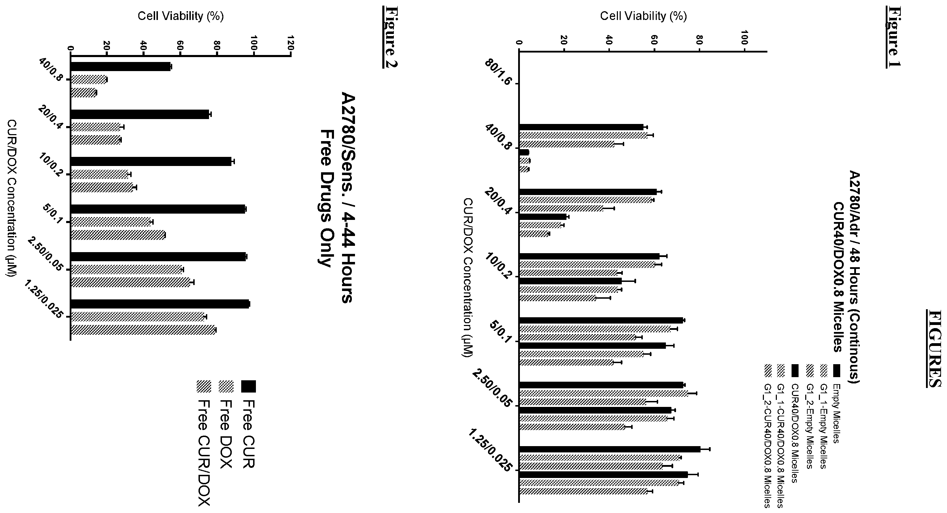

**FIG. 1** depicts, in accordance with the embodiments herein, the efficacy of CUR40/DOX0.8 micelles on Doxorubicin-resistant ovarian carcinoma cell lines, A2780/Adr, after 48 hours of continuous contact. The columns show the cell viability at CUR/DOX concentrations 80/1.6, 40/0.8, 20/0.4, 10/0.2, 5/0.1, 2.5/0.05, 1.25/0.025. Experimental data compares the cell viability of EmptyMicelles, G1_1- EmptyMicelles, G1_2- EmptyMicelles, CUR40/DOX0.8 Micelles, G1_1-CUR40/DOX0.8 Micelles, and G1_2-CUR40/DOX0.8 micelles.
**FIG. 2** depicts, in accordance with the embodiments herein, the efficacy of free CUR, free DOX, and free CUR/DOX on Doxorubicin-sensitive ovarian carcinoma cell lines, A2780/Sens, after 4-44 hours of contact. Experimental data compares the cell viability of free CUR, free DOX, and free CUR/DOX at CUR/DOX (uM) concentrations: 40/0.8, 20/0.4, 10/0.2, 5/0.1, 2.5/0.05, and 1.25/0.025.

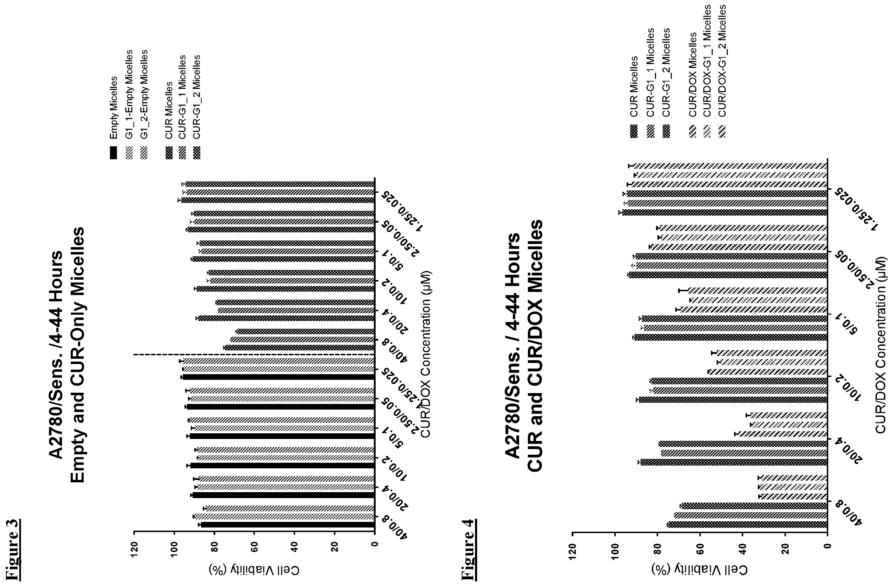

**FIG. 3** depicts, in accordance with the embodiments herein, the efficacy of Empty and CUR-only micelles on Doxorubicin-sensitive ovarian carcinoma cell lines, A2780/Sens, after 4-44 hours of contact. The data compares the cell viability (%) of EmptyMicelles, G1_1-EmptyMicelles, G1_2-EmptyMicelles, CUR Micelles, CUR-G1_1 Micelles and CUR-G1_2 Micelles at CUR/DOX (uM) concentrations 40/0.8, 20/0.4, 10/0.2, 5/0.1, 2.5/0.05, and 1.25/0.025.
**FIG. 4** depicts, in accordance with the embodiments herein, the efficacy of CUR and CUR/DOX micelles on Doxorubicin-sensitive ovarian carcinoma cell lines, A2780/Sens, after 4-44 hours of contact. The columns show the cell viability at CUR/DOX concentrations 40/0.8, 20/0.4, 10/0.2, 5/0.1, 2.5/0.05, and 1.25/0.025. Experimental data compares the cell viability of CUR Micelles, CUR-G1_1 Micelles, CUR-G1 Micelles, CUR/DOX Micelles, CUR/DOX-G1_1 Micelles, and CUR/DOX-G1_2 Micelles.

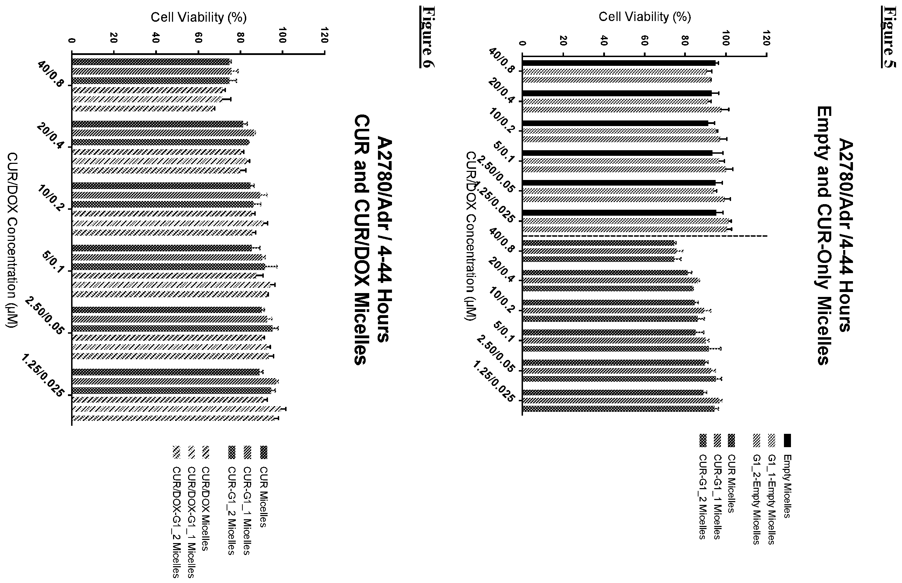

**FIG. 5** depicts, in accordance with the embodiments herein, the efficacy of empty and CUR-only micelles on Doxorubicin-resistant ovarian carcinoma cell lines, A2780/Adr after 4-44 hours of contact. The columns show the cell viability at CUR/DOX (uM) concentrations 40/0.8, 20/0.4, 10/0.2, 5/0.1, 2.5/0.05, and 1.25/0.025. Experimental data compares the cell viability of Empty Micelles, G1_1-Empty Micelles, G1_2-Empty Micelles, CUR Micelles, CUR-G1_1 Micelles, and CUR-G1_2 Micelles.
**FIG. 6** depicts, in accordance with the embodiments herein, the efficacy of CUR and CUR/DOX micelles on Doxorubicin-resistant ovarian carcinoma cell lines, A2780/Adr after 4-44 hours of contact. The columns show the cell viability at CUR/DOX (uM) concentrations 40/0.8, 20/0.4, 10/0.2, 5/0.1, 2.5/0.05, and 1.25/0.025. Experimental data compares the cell viability of CUR Micelles, CUR-G1_1 Micelles, CUR-G1_2 Micelles, CUR/DOX Micelles, CUR/DOX-G1_1 Micelles, and CUR/DOX-G1_2 Micelles.

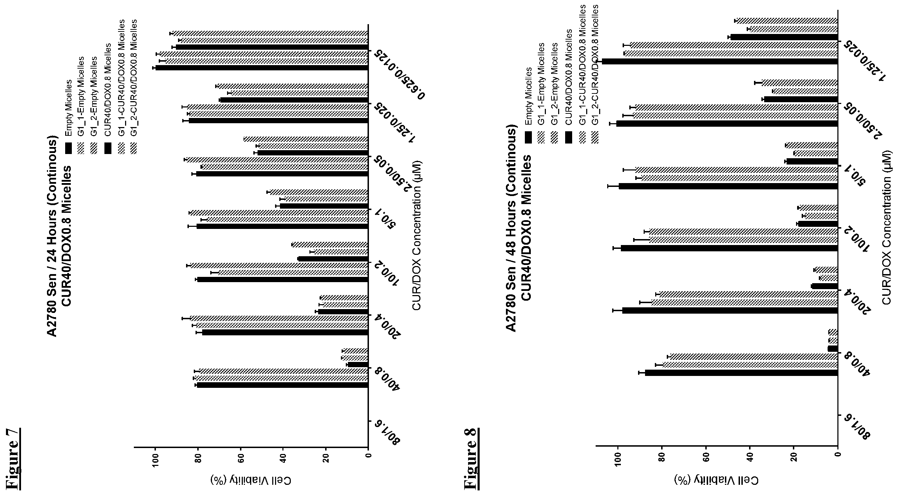

**FIG. 7** depicts, in accordance with the embodiments herein, the efficacy of CUR40/DOX0.8 micelles on Doxorubicin-sensitive ovarian carcinoma cell lines, A2780/Sen after 24 hours of continuous contact. The columns show the cell viability at CUR/DOX (uM) concentrations 80/1.6, 40/0.8, 20/0.4, 10/0.2, 5/0.1, 2.5/0.05, 1.25/0.025, and 0.625/0.0125. Experimental data compares the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles.
**FIG. 8** depicts, in accordance with the embodiments herein, the efficacy of CUR40/DOX0.8 micelles on Doxorubicin-sensitive ovarian carcinoma cell lines, A2780/Sen, after 48 hours of continuous contact. The columns show the cell viability at CUR/DOX (uM) concentrations 80/1.6, 40/0.8, 20/0.4, 10/0.2, 5/0.1, 2.5/0.05, and 1.25/0.025. Experimental data compares the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles.

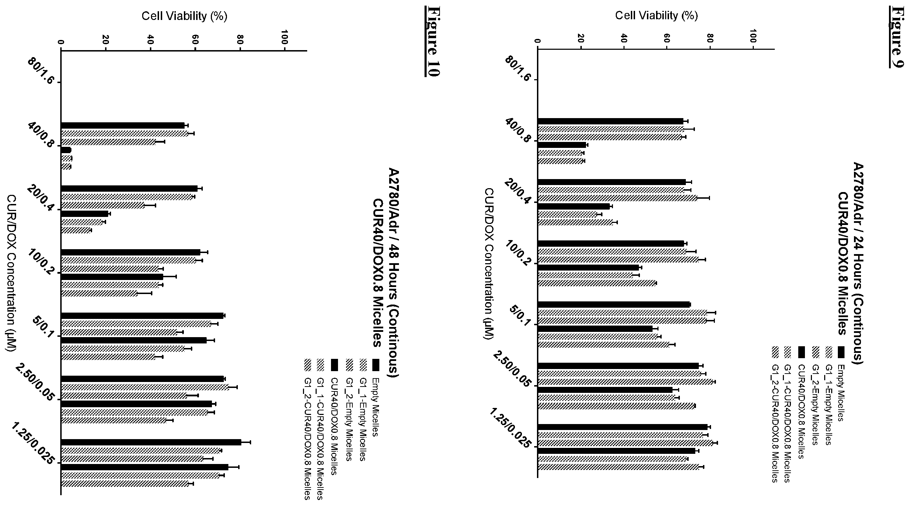

**FIG. 9** depicts, in accordance with the embodiments herein, the efficacy of CUR40/DOX0.8 micelles on Doxorubicin-resistant ovarian carcinoma cell lines, A2780/Adr after 24 hours of continuous contact. The columns show the cell viability at CUR/DOX (uM) concentrations 80/1.6, 40/0.8, 20/0.4, 10/0.2, 5/0.1, 2.5/0.05, and 1.25/0.025. Experimental data compares the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles.
**FIG. 10** depicts, in accordance with the embodiments herein, the efficacy of CUR40/DOX0.8 micelles on Doxorubicin-resistant ovarian carcinoma cell lines, A2780/Adr, after 48 hours of continuous contact. The columns show the cell viability at CUR/DOX (uM) concentrations 80/1.6, 40/0.8, 20/0.4, 10/0.2, 5/0.1, 2.5/0.05, and 1.25/0.025. Experimental data compares the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles.

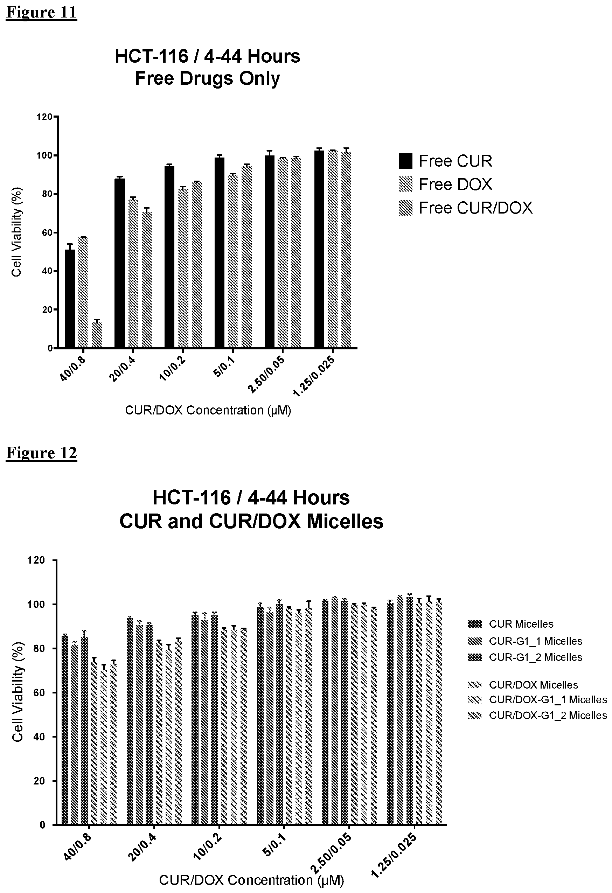

**FIG. 11** depicts, in accordance with the embodiments herein, the efficacy of free CUR, free DOX, and free CUR/DOX on HCT-116 cells after 4-44 hours of contact. The columns illustrate the cell viability (%) at CUR/DOX (uM) concentrations: 40/0.8, 20/0.4, 10/0.2, 5/0.1, 2.5/0.05, and 1.25/0.025.
**FIG. 12** depicts, in accordance with the embodiments herein, the efficacy of CUR and CUR/DOX micelles on HCT-116 cells after 4-44 hours of contact. The columns show the cell viability at CUR/DOX (uM) concentrations 40/0.8, 20/0.4, 10/0.2, 5/0.1, 2.5/0.05, and 1.25/0.025. Experimental data compares the cell viability of CUR Micelles, CUR-G1_1 Micelles, CUR-G1_2 Micelles, CUR/DOX Micelles, CUR/DOX-G1_1 Micelles, and CUR/DOX-G1_2 Micelles.

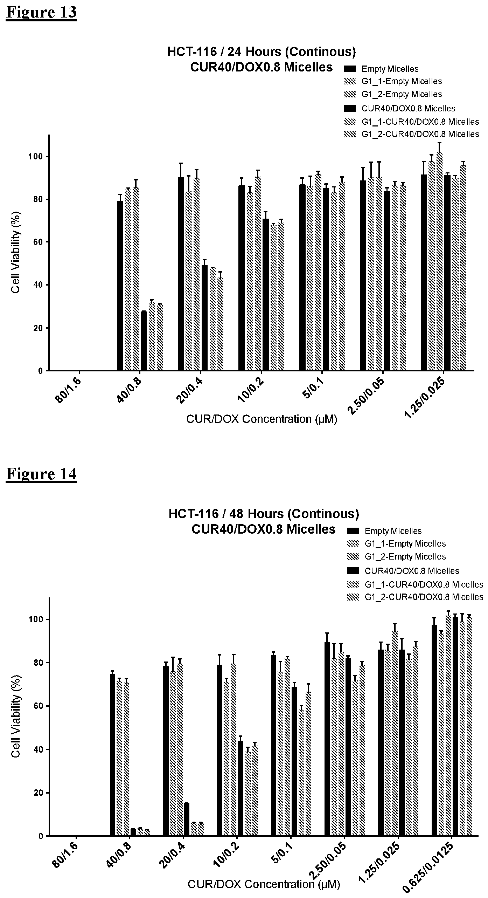

**FIG. 13** depicts, in accordance with the embodiments herein, the efficacy of CUR40/DOX0.8 micelles HCT-116 cells after 24 hours of continuous contact. The columns show the cell viability at CUR/DOX (uM) concentrations 80/1.6, 40/0.8, 20/0.4, 10/0.2, 5/0.1, 2.5/0.05, and 1.25/0.025. Experimental data compares the cell viability (%) of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles.
**FIG. 14** depicts, in accordance with the embodiments herein, the efficacy of CUR40/DOX0.8 micelles on HCT-116 cells after 48 hours of continuous contact. The columns show the cell viability at CUR/DOX (uM) concentrations 80/1.6, 40/0.8, 20/0.4, 10/0.2, 5/0.1, 2.5/0.05, and 1.25/0.025. Experimental data compares the cell viability (%) of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles.

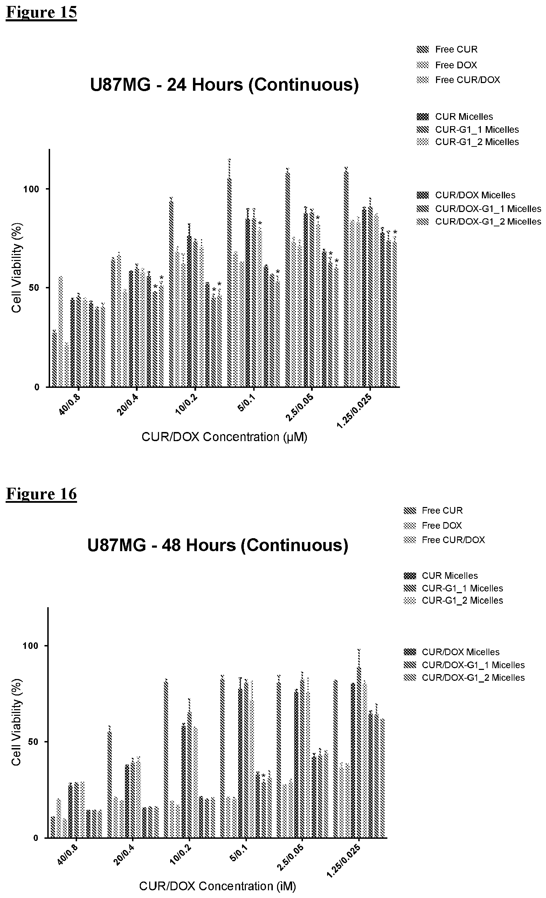

**FIG. 15** depicts, in accordance with the embodiments herein, the efficacy of free drugs and micelles on U87MG cells after 24 hours of continuous contact. The columns show the cell viability at CUR/DOX (uM) concentrations 40/0.8, 20/0.4, 10/0.2, 5/0.1, 2.5/0.05, and 1.25/0.025. Experimental data compares the cell viability (%) of free CUR, free DOX, free CUR/DOX, CUR Micelles, CUR-G1_1 Micelles, CUR-G1_2 Micelles, CUR/DOX Micelles, CUR/DOX-G1_1 Micelles, and CUR/DOX-G1_2 Micelles.
**FIG. 16** depicts, in accordance with the embodiments herein, the efficacy of free drugs and micelles on U87MG cells after 48 hours of continuous contact. The columns show the cell viability (%) at CUR/DOX (uM) concentrations 40/0.8, 20/0.4, 10/0.2, 5/0.1, 2.5/0.05, and 1.25/0.025. Experimental data compares the cell viability of free CUR, free DOX, free CUR/DOX, CUR Micelles, CUR-G1_1 Micelles, CUR-G1_2 Micelles, CUR/DOX Micelles, CUR/DOX-G1_1 Micelles, and CUR/DOX-G1_2 Micelles.

### DETAILED DESCRIPTION

Unless defined otherwise, technical and scientific terms used herein have the same meaning as commonly understood by one of ordinary skill in the art to which this invention belongs. Hornyak, et al., Introduction to Nanoscience and Nanotechnology, CRC Press (2008); Singleton et al., Dictionary of Microbiology and Molecular Biology 3rd ed., J. Wiley & Sons (New York, NY 2001); March, Advanced Organic Chemistry Reactions, Mechanisms and Structure 7th ed., J. Wiley & Sons (New York, NY 2013); and Sambrook and Russel, Molecular Cloning: A Laboratory Manual 4th ed., Cold Spring Harbor Laboratory Press (Cold Spring Harbor, NY 2012), provide one skilled in the art with a general guide to many of the terms used in the present application. One skilled in the art will recognize many methods and materials similar or equivalent to those described herein, which could be used in the practice of the present invention. Indeed, the present invention is in no way limited to the methods and materials described.

As used herein, "HCT-116" refers to a colon tumor cell line. As used herein, "A2780" refers to an ovarian cancer cell line. As used herein, "U87MG" or "U87" refers to a brain cancer cell line.

As used herein, the abbreviation of "CUR" refers to curcumin. As used herein, the term "DOX" refers to doxorubicin.

As used herein, the terms "glut-1," "GLUT-1," "G1," or "glut1," used interchangeably, refers to a glucose transporter antibody. The term scFv, as used herein, refers to single chain variable fragment. Thus, the terms GLUT-1_1 and/or GLUT1_2 scFv refers to a single chain variable fragment of the GLUT-1 antibody.

As described herein, the inventor has recognized that effective cancer treatment would benefit from attacking the cancer early, as well as attacking aggressively. This could come in the form of administering a combination of drugs for treatment, as the odds of a single cell being resistant to a larger quantity of drugs are lower. Additionally, an effective cancer treatment could also potentially bypass the diversity of cancer cells by targeting processes that cancer cells rely on for their very growth. One such process is tumors' reliance on producing and processing sugar for its cell growth.

As described herein, the inventor has developed various compositions and methods for the treatment of cancers and associated conditions. In accordance with embodiments further described herein, the inventor developed and optimized cancer therapeutic compositions and methods for effective delivery and minimized toxicity. For example, in one embodiment, utilizing a single chain antibody allows a more efficient delivery in conjunction with various embodiments herein. In accordance with various embodiments further disclosed herein, the inventor also attached curcumin to a targeted micelle. By attaching curcumin to a targeted micelle, treatment was administered at a significantly lower dosage, thus reducing toxicity while effectively inhibiting tumor growth. As compared to the liposomal forms of both doxorubicin and curcumin, a micellar preparation is of significantly smaller molecular size (10-20 nm vs. 80-150 nm liposomes) resulting in improved tumor mass penetration from the vascular bed, thus creating a more effective cancer therapeutic.

In one embodiment, disclosed herein are compositions comprising a micelle construct; an antibody single chain fragment variable (scFv); a NF-kb inhibitor; and a topoisomerase II inhibitor. In one embodiment, the scFv is conjugated to the micelle construct. In one embodiment, the micelle construct is conjugated to the NF-kb inhibitor. In one embodiment, the micelle construct is conjugated to the NF-kb inhibitor and the topoisomerase II inhibitor. In one embodiment, the NF-kb inhibitor is curcumin or a pharmaceutical equivalent, analog, derivative, and/or salt thereof. In one embodiment, the topoisomerase II inhibitor is dox or a pharmaceutical equivalent, analog, derivative, and/or salt thereof. In one embodiment, the scFv is a glut-1_1 or glut-1_2 scFv. In one embodiment, the micelle construct is targeted to bind to glut-1_1 and/or glut-1_2 scFv. In one embodiment, the composition forms a targeted micelle. In one embodiment, the molecular size of the targeted micelle is 30 nm or less. In one embodiment, the composition further comprises a pharmaceutically acceptable carrier.

In an example, disclosed herein are methods of delivering a drug molecule to a tumor site of a subject comprising: attaching the drug molecule to a targeted micelle, wherein the targeted micelle comprises a micelle construct and an antibody single chain fragment variable (scFv); and delivering the drug molecule attached to the targeted micelle to the tumor site through intracellular delivery. In one embodiment, the drug molecule comprises dox and/or curcumin or a pharmaceutical equivalent, analog, derivative, and/or salt thereof. In one embodiment, the molecular size of the micelle is 30 nm or less. In one embodiment, the tumor is a doxorubicin-resistant tumor. In one embodiment, the subject is human.

In another example, disclosed herein are methods of treating cancer comprising administering to a subject in need thereof, a therapeutically effective dosage of a composition comprising a micelle construct, an antibody single chain fragment variable (scFv), a NF-kb inhibitor, and a topoisomerase II inhibitor. In one example, the composition is administered to the subject intravenously. In one embodiment, the subject is human. In one embodiment, the cancer is brain cancer. In one embodiment, the cancer is ovarian cancer. In one embodiment, the cancer is colon cancer. In one embodiment, the cancer is a doxorubicin-resistant cancer. In one embodiment, the molecular size of the targeted micelle is 30 nm or less.

In another example, disclosed herein are methods of inhibiting cell growth of a tumor cell, comprising: providing a composition comprising a micelle construct, an antibody single chain fragment variable (scFv), a NF-kb inhibitor and a topoisomerase II inhibitor; and inhibiting cell growth by administering a therapeutically effective dosage of the composition to the tumor cell. In one embodiment, the topoisomerase inhibitor comprises Dox. In one embodiment, the NF-kB inhibitor comprises curcumin. In one embodiment, the tumor cell is a brain cancer cell, a ovarian cancer cell, and/or colon cancer cell.

In another embodiment, disclosed herein are nanoconjugates comprising: a micelle construct; an antibody single chain fragment variable (scFv); a NF-kb inhibitor; and a topoisomerase II inhibitor; and wherein the micelle construct, the antibody single chain fragment variable (scFv), the NF-kb inhibitor, and the topoisomerase II inhibitor are conjugated to one another. In one embodiment, the nanoconjugate is between 20 nm and 50 nm. In one embodiment, the nanoconjugate is between 10 nm and 20 nm. In one embodiment, the nanoconjugate is less than 20 nm. In one embodiment, the nanoconjugate is enclosed by a micelle.

### EXAMPLES

### Example 1

### Materials

1,2-Distearoyl-*sn*-glycero-3-phosphoethanolamine-N-[methoxy (polyethylene glycol)-2000] (PEG-PE) was from Avanti Polar Lipids (Alabaster, AL, USA). *pNP*-PEG₃₄₀₀-*pNP* was purchased from Laysan Bio (Arab, AL). Curcumin (CUR), vitamin E and triethylamine (TEA) were purchased from Sigma (St. Louis, MO, USA). Doxorubicin HCl (DOX) was from LC Laboratories (Woburn, MA). Accumax was from Innovative Cell Technologies, Inc. (San Diego, CA). The CellTiter-Glo luminescent cell viability assay kit was from Promega (Fitchburg, WI). All other reagents were of analytical grade. DOX free base was obtained by incubating DOX HCl (in methanol, 0.5 mg/ml) with 5-fold molar excess of TEA overnight. pNP-PEG₃₄₀₀-PE was synthesized in-house.

### Example 2

### Micelle Preparation

DOX and/or CUR drug-loaded mixed micelles were prepared by the thin film hydration method. CUR (1 mg/ml in 0.1% acetic acid-methanol solution) and/or DOX free base (0.5 mg/ml in methanol solution) were added to PEG₂₀₀₀-PE solution in chloroform. Initial drug amounts were adjusted after the formulation optimization studies to result in CUR:DOX ratio of 32 (w/w). The organic solvents were removed by the rotary evaporation and a thin film of drugs/micelle-forming material mixture was formed. This film was further dried overnight in freeze-dryer to remove any residuals of organic solvents (Freezone 4.5 Freeze Dry System, Labconco, Kansas City, MO). Drug-loaded micelles were formed by resuspending the film in phosphate buffered saline (PBS) pH 7.2, to give the final concentration of micelle forming materials of 5 mM in all formulations. The micelle formulations were dialyzed 1h against PBS pH 7.2 to remove excess of TEA. Excess non-incorporated drugs were separated by filtration through a 0.2 µm syringe filter.

To obtain targeted micelles, GLUT-1 scFv was reacted with *pNP*-PEG₃₄₀₀-PE. Briefly, required amount of *pNP*-PEG₃₄₀₀-PE in chloroform was added into the round bottom flask and polymer film was formed after removing the solvent by rotary evaporation followed by drying under vacuum. The film was hydrated and vortexed first with citrate-buffered saline (CBS) pH 5.0 to prevent early hydrolysis of *pNP* distal groups. After forming micelles, GLUT-1 scFv solution in PBS (pH 7.4) was added to the mixture, and the pH was adjusted to 8.2 with NaOH. Molar ratio of *pNP* groups to scFv was kept at 40:1. Reaction time was overnight at 4 °C to allow sufficient conjugation and the complete hydrolysis of the unreacted *pNP* groups at the higher pH. Targeted-micelles were then dialyzed using a 50,000 MWCO cellulose ester membrane against PBS (pH 7.4) for 4 hours at 4 °C to ensure the complete removal of the unconjugated scFv. Conjugation efficiency of GLUT-1 scFv was measured using a micro BCA kit (Pierce, Rockford, IL) according to the manufacturer's instructions. GLUT-1 scFv conjugated micelles were mixed and co-incubated with drug loaded PEG-PE micelles for 4 hours at room temperature. The final GLUT-1 scFv mol ratio in the micelles was adjusted to 0.05%.

### Example 3

### GLUT-1 scFv Modified Micelle Cytotoxicity on Ovarian Cancer Cell Line

For cytotoxicity evaluations, A2780 human ovarian carcinoma (ATCC) and its Doxorubicin resistant derivative A2780/Adr (ECACC) were used. A2780 cells were cultured in RPMI medium supplemented with 10% FBS and 1% penicillin-streptomycin. Drug resistant ovarian cancer cells were grown in same medium with the addition of 100 nM DOX.

The cells were seeded in 96-well plates at a density of 3000 cells/well or 5,000 cells/well for 48 or 24 hours treatment groups, respectively. The cells were treated with free drugs or micelle formulations containing 40 µM CUR and 0.8 µM DOX in serum complete RPMI medium. The cells were incubated 24h and 48h continuously in the continuous treatment groups. Additionally, in another treatment regimen the cells were incubated with drugs/micelles for 4 hours then washed and further incubated for 44h. At the end of treatment times, the wells were washed twice with medium and then incubated with 50 µl medium containing 20% (v/v) CellTiter-Blue reagent. The fluorescent emission values were measured and % cell viability was calculated by using PBS treated cells as the control group.

### Example 4

### 3D Glioblastoma Spheroids Preparation and Cytotoxicity Evaluation of Micelles

The U87MG cells were cultured in DMEM medium supplemented with 10% FBS and 1% penicillin-streptomycin. For monolayer cytotoxicity experiments 3000 or 5000 cells were seeded into the each well of 96-well plate for 48h and 24h continuous treatment times, respectively. After overnight incubation of the plates, the cells were treated with CUR and/or DOX as free drugs or micellar formulations. CellTiter-Blue was used as the cytotoxicity evaluation method as described above.

For 3D cancer cell spheroid preparation, liquid overlay method was used. Briefly, serum free DMEM medium with 1.5% (w/v) agar was prepared and sterilized. 50 µL of the agar solution were added to the bottom of each well of the 96 well plates to prevent cell adhesion onto the well surface. Plates were allowed to cool down for 45 minutes before use. U87MG cells were trypsinized, counted and then seeded at the density of 10,000 cells/well. Plates were centrifuged for 15 min at 1,500 rcf at 24°C. Spheroid formation was continuously followed using Nikon Eclipse E400 microscope (Nikon Inc., Melville, NY) with the Spot Insight camera and Spot Advanced software (Spot Imaging, Sterling Heights, MI). When the spheroids are formed and dense after 5 days, they were treated with the formulations. Cellular viability of the spheroids was determined after 48h treatment. After the treatment with different formulations, drug concentrations and combinations, all the media from the corresponding wells along with the spheroids were collected and placed in a centrifuge tube. Five spheroids were collected as one replicate to increase the sensitivity. The spheroids were washed two times with PBS. Following removal of the remaining PBS, AccuMax® cell detachment solution was added and tubes were incubated for 10 minutes at 37°C on the horizontal shaker with occasional pipetting. After dispersing of the spheroids into single cells, FBS was added into the tubes. Obtained cells were centrifuged and supernatants were discarded. Complete DMEM and CellTiter-Glo reagent at 1:1 volume ratio was added into the tubes and cells were incubated for 20 minutes for complete cell lysis. Luminescence was measured using multiplate reader by transferring 100 µL of the solution into 96 well black-walled plates.

### Example 5

### Compositions and methods

As described herein, the inventor has developed various compositions and methods for the treatment of cancers and associated conditions. In one example, the present disclosure provides a method of treating cancer and/or inhibiting growth in a tumor cell in a subject, by providing a composition comprising a micelle targeted by a glut-1 receptor antibody and attached to one or more curcumin and/or dox molecules, and administering a therapeutically effective dosage to the subject. In another example, the composition is administered to the subject intravenously. In another embodiment the glut-1 receptor antibody is a single chain antibody. In another embodiment, the glut-1 receptor antibody is GLUT-1_1 and/or GLUT1_2 scFV. In another embodiment, the tumor cell is an ovarian tumor. In another embodiment, the tumor cell is a brain tumor. In another embodiment, the tumor cell is A2780. In another embodiment, the tumor cell is U87MG.

In accordance with embodiments further described herein, the inventor developed and optimized cancer therapeutic compositions and methods for effective delivery and minimized toxicity. For example, in one embodiment, utilizing a single chain antibody allowed a more efficient delivery in conjunction with various embodiments herein. Also, for example, by utilizing dox attached to a targeted micelle as a lipid-based delivery vehicle, rather than liposomal dox, or just dox, for example, the inventor created a cancer treatment with significantly high penetration of tumor mass. In addition to creating high tumor mass penetration, administering a composition comprising a dox attached to a targeted micelle optimized intracellular delivery of dox within the tumor cell itself. Because dox acts as a weakly basic compound, if it enters a low pH environment or the lysosome of a tumor cell for example, the dox can lose much of its effectiveness for inhibiting tumor cell growth. By administering dox attached to a targeted micelle, rather than administering liposomal dox for example, the inventor enabled the dox to instead enter the cytoplasm, thus optimizing intracellular delivery. Additionally, dox can have high toxicity which can thus limit its practical application in vivo and usefulness as a cancer treatment for human subjects. In contrast, the inventor administered dox attached to a targeted micelle, resulting in further optimization of its effectiveness as a cancer therapeutic.

In accordance with various embodiments further disclosed herein, the inventor also attached curcumin to a targeted micelle. Due to its non-soluble properties, if administered directly, curcumin must be administered at high concentrations to be effective inhibiting tumor growth. However, at those same high concentrations, curcumin results in high toxicity, thus making it an impractical and ineffective cancer treatment in vivo, and particularly difficult for use in human patients. In accordance with an embodiment herein, by attaching curcumin to a targeted micelle, treatment can be administered at a significantly lower dosage, thus reducing toxicity while effectively inhibiting tumor growth.

As compared to the liposomal forms of both doxorubicin and curcumin, a micellar preparation is of significantly smaller molecular size (10-20 nm vs. 80-150 nm liposomes) resulting in improved tumor mass penetration from the vascular bed, thus creating a more effective cancer therapeutic. Additionally, in accordance with an embodiment herein, the addition of the Glut-1 Ab to the micelle greatly increased its therapeutic efficacy over the untargeted micelle preparations through improved intracellular delivery of its contents. Glut-1 presents an attractive extracellular target since it is one of the main glucose transporters involved in tumor glucose uptake. Solid tumors can take up glucose at much higher rates than do normal cells. Glycolysis represents a main source of energy and carbon building blocks for growing tumors. Thus, in accordance with an embodiment herein, a micelle construct with a Glut-1 Ab is an effective cancer therapeutic, as glut-1 overexpression will persist even in the face of tumor phenotypic evolution, and it will be difficult for tumors to mutate away from glut-1 overexpression and still retain their high growth potential. In regard to using glut-1 as the tumor targeting entity, by binding to the tumor membrane-overexpressed glut-1, various embodiments of therapeutic micelles described herein get endocytosed into the cytoplasm rather than the low-pH lysosome, thereby increasing the therapeutic efficacy of doxorubicin (which has much lower activity at low pH).

Since the tumor lines used in these experiments were doxorubicin-resistant, it was critical that curcumin delivery occurred contemporaneously with doxorubicin exposure in order for it to exert its tumoricidal effect. As NFκB overexpression and its attendant apoptosis- and chemotherapy-resistance are present in advanced cancers, the inventor designed various embodiments herein to include an NFκB inhibitor (for example, curcumin) in order to unlock the cidal effect of doxorubicin. In one embodiment, the present invention is a tumor-targeted micelle containing a tumor-cidal agent coupled with an apoptosis inhibitor with significant in vivo tumor inhibitory effect and clear applicability to human cancer therapeutics.

### Example 6

### Efficacy on chemo-resistant ovarian carcinoma cells (A2780/Adr)

In one embodiment, the inventor has demonstrated the efficacy of the compounds and micelles disclosed herein in studies on Doxorubicin-resistant ovarian carcinoma cells. At 20/0.4uM CUR/DOX concentration, the enhancing effect of the Glut1 antibody targeting is clearly visible with reduced tumor cell viability.

As illustrated in Fig. 1, the efficacy of CUR40/DOX0.8 micelles on Doxorubicin-resistant ovarian carcinoma cell lines, A2780/Adr, after 48 hours of continuous contact has been determined. The columns show the cell viability at CUR/DOX concentrations 80/1.6, 40/0.8, 20/0.4, 10/0.2, 5/0.1, 2.5/0.05, 1.25/0.025. Experimental data comparing the cell viability of EmptyMicelles, G1_1- EmptyMicelles, G1_2- EmptyMicelles, CUR40/DOX0.8 Micelles, G1_1-CUR40/DOX0.8 Micelles, and G1_2-CUR40/DOX0.8 micelles is shown.

As illustrated in Fig. 2, the efficacy of free CUR, free DOX, and free CUR/DOX on Doxorubicin-sensitive ovarian carcinoma cell lines, A2780/Sens, after 4-44 hours of contact has been determined. Experimental data comparing the cell viability of free CUR, free DOX, and free CUR/DOX at CUR/DOX (uM) concentrations: 40/0.8, 20/0.4, 10/0.2, 5/0.1, 2.5/0.05, and 1.25/0.025 is shown.

As illustrated in Fig. 3, the efficacy of empty and CUR-only micelles on Doxorubicin-sensitive ovarian carcinoma cell lines, A2780/Sens, after 4-44 hours of contact has been determined. The data comparing the cell viability (%) of EmptyMicelles, G1_1-EmptyMicelles, G1_2-EmptyMicelles, CUR Micelles, CUR-G1_1 Micelles and CUR-G1_2 Micelles at CUR/DOX (uM) concentrations 40/0.8, 20/0.4, 10/0.2, 5/0.1, 2.5/0.05, and 1.25/0.025 is shown.

As illustrated in Fig. 4, the efficacy of CUR and CUR/DOX micelles on Doxorubicin-sensitive ovarian carcinoma cell lines, A2780/Sens, after 4-44 hours of contact has been determined. The columns show the cell viability at CUR/DOX concentrations 40/0.8, 20/0.4, 10/0.2, 5/0.1, 2.5/0.05, and 1.25/0.025. Experimental data comparing the cell viability of CUR Micelles, CUR-G1_1 Micelles, CUR-G1 Micelles, CUR/DOX Micelles, CUR/DOX-G1_1 Micelles, and CUR/DOX-G1_2 Micelles is shown.

As illustrated in Fig. 5, the efficacy of empty and CUR-only micelles on Doxorubicin-resistant ovarian carcinoma cell lines, A2780/Adr after 4-44 hours of contact has been determined. The columns show the cell viability at CUR/DOX (uM) concentrations 40/0.8, 20/0.4, 10/0.2, 5/0.1, 2.5/0.05, and 1.25/0.025. Experimental data comparing the cell viability of Empty Micelles, G1_1-Empty Micelles, G1_2-Empty Micelles, CUR Micelles, CUR-G1_1 Micelles, and CUR-G1_2 Micelles is shown.

As illustrated in Fig. 6, the efficacy of CUR and CUR/DOX micelles on Doxorubicin-resistant ovarian carcinoma cell lines, A2780/Adr after 4-44 hours of contact has been determined. The columns show the cell viability at CUR/DOX (uM) concentrations 40/0.8, 20/0.4, 10/0.2, 5/0.1, 2.5/0.05, and 1.25/0.025. Experimental data comparing the cell viability of CUR Micelles, CUR-G1_1 Micelles, CUR-G1_2 Micelles, CUR/DOX Micelles, CUR/DOX-G1_1 Micelles, and CUR/DOX-G1_2 Micelles is shown.

As illustrated in Fig. 7, the efficacy of CUR40/DOX0.8 micelles on Doxorubicin-sensitive ovarian carcinoma cell lines, A2780/Sen after 24 hours of continuous contact has been determined. The columns show the cell viability at CUR/DOX (uM) concentrations 80/1.6, 40/0.8, 20/0.4, 10/0.2, 5/0.1, 2.5/0.05, 1.25/0.025, and 0.625/0.0125. Experimental data comparing the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles is shown.

As illustrated in Fig. 8, the efficacy of CUR40/DOX0.8 micelles on Doxorubicin-sensitive ovarian carcinoma cell lines, A2780/Sen, after 48 hours of continuous contact has been determined. The columns show the cell viability at CUR/DOX (uM) concentrations 80/1.6, 40/0.8, 20/0.4, 10/0.2, 5/0.1, 2.5/0.05, and 1.25/0.025. Experimental data comparing the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles is shown.

As illustrated in Fig. 9, the efficacy of CUR40/DOX0.8 micelles on Doxorubicin - resistant ovarian carcinoma cell lines, A2780/Adr after 24 hours of continuous contact has been determined. The columns show the cell viability at CUR/DOX (uM) concentrations 80/1.6, 40/0.8, 20/0.4, 10/0.2, 5/0.1, 2.5/0.05, and 1.25/0.025. Experimental data comparing the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles is shown.

As illustrated in Fig. 10, the efficacy of CUR40/DOX0.8 micelles on Doxorubicin resistant ovarian carcinoma cell lines, A2780/Adr, after 48 hours of continuous contact has been determined. The columns show the cell viability at CUR/DOX (uM) concentrations 80/1.6, 40/0.8, 20/0.4, 10/0.2, 5/0.1, 2.5/0.05, and 1.25/0.025. Experimental data comparing the cell viability of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles is shown.

### Example 7

### Efficacy on colon cancer cell lines

In one embodiment, the inventor has demonstrated the efficacy of the compounds and micelles disclosed herein on human colon carcinoma cell line HCT-116.

As illustrated in Fig. 11, the efficacy of free CUR, free DOX, and free CUR/DOX on HCT-116 cells after 4-44 hours of contact has been determined. The columns illustrate the cell viability (%) at CUR/DOX (uM) concentrations: 40/0.8, 20/0.4, 10/0.2, 5/0.1, 2.5/0.05, and 1.25/0.025.

As illustrated in Fig. 12, the efficacy of CUR and CUR/DOX micelles on HCT-116 cells after 4-44 hours of contact has been determined. The columns show the cell viability at CUR/DOX (uM) concentrations 40/0.8, 20/0.4, 10/0.2, 5/0.1, 2.5/0.05, and 1.25/0.025. Experimental data comparing the cell viability of CUR Micelles, CUR-G1_1 Micelles, CUR-G1_2 Micelles, CUR/DOX Micelles, CUR/DOX-G1_1 Micelles, and CUR/DOX-G1_2 Micelles is shown.

As illustrated in Fig. 13, the efficacy of CUR40/DOX0.8 micelles HCT-116 cells after 24 hours of continuous contact has been determined. The columns show the cell viability at CUR/DOX (uM) concentrations 80/1.6, 40/0.8, 20/0.4, 10/0.2, 5/0.1, 2.5/0.05, and 1.25/0.025. Experimental data comparing the cell viability (%) of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles is shown.

As illustrated in Fig. 14, the efficacy of CUR40/DOX0.8 micelles on HCT-116 cells after 48 hours of continuous contact has been determined. The columns show the cell viability at CUR/DOX (uM) concentrations 80/1.6, 40/0.8, 20/0.4, 10/0.2, 5/0.1, 2.5/0.05, and 1.25/0.025. Experimental data comparing the cell viability (%) of Empty Micelles, G1_1-Empty Micelles, G1_2-Empty Micelles, CUR40/DOX0.8 Micelles, G1_1-CUR40/DOX0.8 Micelles, and G1_2-CUR40/DOX0.8 Micelles is shown.

### Example 8

### Efficacy on brain cancer cell line

In one embodiment, the efficacy of the compounds and micelles disclosed herein has been demonstrated on human primary glioblastoma cell line U87MG. GLUT 1_1 and GLUT 1_2 scFv are two anti-Glutl scFv's that was produced. The experiments were performed with U87MG monolayer treated with micelles. So far, this cell line was more responsive line to GLUT 1_1 and GLUT 1_2 scFv. 48 hours of contact was too much for this cell line because the cytotoxicity effect was saturated with drugs. However for 24 hours, the row and column effects were significant means that there was a dose response and also the antibody targeting effect. Significant difference corresponded to difference between non-targeted micelles to GLUT 1_1 or GLUT 1_2 targeted micelles, two-way ANOVA, P<0.05. In accordance with various embodiments, administration to U87MGs responded well to GLUT 1_2.

As illustrated in Fig. 15, the efficacy of free drugs and micelles on U87MG cells after 24 hours of continuous contact has been determined. The columns show the cell viability at CUR/DOX (uM) concentrations 40/0.8, 20/0.4, 10/0.2, 5/0.1, 2.5/0.05, and 1.25/0.025. Experimental data comparing the cell viability (%) of free CUR, free DOX, free CUR/DOX, CUR Micelles, CUR-G1_1 Micelles, CUR-G1_2 Micelles, CUR/DOX Micelles, CUR/DOX-G1_1 Micelles, and CUR/DOX-G1_2 Micelles is shown.

As illustrated in Fig. 16, the efficacy of free drugs and micelles on U87MG cells after 48 hours of continuous contact has been determined. The columns show the cell viability (%) at CUR/DOX (uM) concentrations 40/0.8, 20/0.4, 10/0.2, 5/0.1, 2.5/0.05, and 1.25/0.025. Experimental data comparing the cell viability of free CUR, free DOX, free CUR/DOX, CUR Micelles, CUR-G1_1 Micelles, CUR-G1_2 Micelles, CUR/DOX Micelles, CUR/DOX-G1_1 Micelles, and CUR/DOX-G1_2 Micelles is shown.

The various methods and techniques described above provide a number of ways to carry out the invention.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a," "an," and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

## Claims

1. A composition comprising:
a. a micelle construct;
b. an antibody single chain fragment variable (scFv);
c. a NF-kb inhibitor, which is curcumin and/or salt thereof; and
d. a topoisomerase II inhibitor, which is doxorubicin and/or salt thereof.

2. The composition of claim 1, wherein;
i) the scFv is conjugated to the micelle construct;
ii) the micelle construct is conjugated to the NF-kb inhibitor; or
iii) the micelle construct is conjugated to the NF-kb inhibitor and the topoisomerase II inhibitor.

3. The composition of claim 1, wherein the scFv is a glut-1_1 or glut-1_2 scFv.

4. The composition of claim 1, wherein the micelle construct is targeted to bind to glut-1_1 and/or glut-1_2 scFv.

5. The composition of claim 1, wherein the composition forms a targeted micelle, optionally wherein the molecular size of the targeted micelle is 30 nm or less.

6. The composition of claim 1, further comprising a pharmaceutically acceptable carrier.

7. The composition according to any one of claims 1 to 6, for use in therapy.

8. The composition according to any one of claims 1 to 6, for use in the treatment of cancer, or for use in the delivery of a drug molecule to a tumor site.

9. The composition for use according to claim 8, wherein the cancer is brain cancer, ovarian cancer or colon cancer.

10. The composition for use according to claim 8, wherein the cancer is a doxorubicin-resistant cancer.

11. A nanoconjugate, comprising:
a. a micelle construct;
b. an antibody single chain fragment variable (scFv);
c. an NF-kb inhibitor, which is curcumin and/or salt thereof; and
d. a topoisomerase II inhibitor which is doxorubicin and/or salt thereof;
wherein the micelle construct, the antibody single chain fragment variable (scFv), the NF-kb inhibitor, and the topoisomerase II inhibitor are conjugated to one another.

12. The nanoconjugate of claim 11, wherein the nanoconjugate has a molecular size between 20 nm and 50 nm.

13. The nanoconjugate of claim 11, wherein the nanoconjugate has a molecular size between 10 nm and 20 nm.

14. The nanoconjugate of claim 11, wherein the nanoconjugate has a molecular size less than 20 nm.

15. The nanoconjugate of claim 11, wherein the nanoconjugate is enclosed by a micelle.

## Patentansprüche

1. Zusammensetzung, umfassend:
a. ein Micellenkonstrukt;
b. ein scFV (Single-chain variable Fragment)-Antikörper;
c. ein NF-kb-Hemmer, bei dem es sich um Curcumin und/oder ein Salz davon handelt; und
d. ein Topoisomerase-II-Hemmer, bei dem es sich um Doxorubicin und/oder ein Salz davon handelt.

2. Zusammensetzung nach Anspruch 1, wobei:
i) das scFv an das Micellenkonstrukt konjugiert ist;
ii) das Micellenkonstrukt an den NF-kb-Hemmer konjugiert ist; oder
iii) das Micellenkonstrukt an den NF-kb-Hemmer und den Topoisomerase-II-Hemmer konjugiert ist.

3. Zusammensetzung nach Anspruch 1, wobei das scFv ein glut-1_1 oder glut-1_2 scFv ist.

4. Zusammensetzung nach Anspruch 1, wobei das Micellenkonstrukt als Ziel eine Bindung an glut-1_1 und/oder glut-1_2 scFv hat.

5. Zusammensetzung nach Anspruch 1, wobei die Zusammensetzung eine Ziel-Micelle bildet, wobei die Molekülgröße der Ziel-Micelle 30 nm oder kleiner ist.

6. Zusammensetzung nach Anspruch 1, die ferner einen pharmazeutisch akzeptablen Träger umfasst.

7. Zusammensetzung nach einem der Ansprüche 1 bis 6 zum Einsatz in der Therapie.

8. Zusammensetzung nach einem der Ansprüche 1 bis 6 zum Einsatz in der Behandlung von Krebs oder zum Einsatz in der Zufuhr eines Arzneimittelmoleküls an eine Tumorstelle.

9. Zusammensetzung zur Verwendung nach Anspruch 8, wobei es sich bei dem Krebs um Hirnkrebs, Eierstockkrebs oder Kolonkrebs handelt.

10. Zusammensetzung zur Verwendung nach Anspruch 8, wobei es sich bei dem Krebs um einen Doxorubicin-resistenten Krebs handelt.

11. Nanokonjugat, umfassend:
a. ein Micellenkonstrukt;
b. ein scFV (Single-chain variable Fragment)-Antikörper;
c. ein NF-kb-Hemmer, bei dem es sich um Curcumin und/oder ein Salz davon handelt; und
d. ein Topoisomerase-II-Hemmer, bei dem es sich um Doxorubicin und/oder ein Salz davon handelt;
wobei das Micellenkonstrukt, der scFV (Single-chain variable Fragment)-Antikörper, der NF-kb-Hemmer und der Topoisomerase-II-Hemmer aneinander konjugiert sind.

12. Nanokonjugat nach Anspruch 11, wobei das Nanokonjugat eine Molekülgröße zwischen 20 nm und 50 nm aufweist.

13. Nanokonjugat nach Anspruch 11, wobei das Nanokonjugat eine Molekülgröße zwischen 10 nm und 20 nm aufweist.

14. Nanokonjugat nach Anspruch 11, wobei das Nanokonjugat eine Molekülgröße von weniger als 20 nm aufweist.

15. Nanokonjugat nach Anspruch 11, wobei das Nanokonjugat durch eine Micelle umschlossen ist.

## Revendications

1. Composition comprenant :
a. une construction de micelle ;
b. un anticorps à chaîne unique à fragment variable (scFv) ;
c. un inhibiteur de NF-kb, qui est la curcumine et/ou un sel de celle-ci ; et
d. un inhibiteur de topoisomérase II, qui est la doxorubicine et/ou un sel de celle-ci.

2. Composition de la revendication 1, dans laquelle :
i) le scFv est conjugué à la construction de micelle ;
ii) la construction de micelle est conjuguée à l'inhibiteur de NF-kb ; ou
iii) la construction de micelle est conjuguée à l'inhibiteur de NF-kb et à l'inhibiteur de topoisomérase II.

3. Composition de la revendication 1, dans laquelle le scFv est un scFv glut-1_1 ou glut-1_2.

4. Composition de la revendication 1, dans laquelle la construction de micelle est ciblée pour se lier au scFv glut-1_1 et/ou glut-1_2.

5. Composition de la revendication 1, dans laquelle la composition forme une micelle ciblée, éventuellement dans laquelle la taille moléculaire de la micelle ciblée est de 30 nm ou moins.

6. Composition de la revendication 1, comprenant en outre un véhicule pharmaceutiquement acceptable.

7. Composition selon l'une quelconque des revendications 1 à 6, destinée à une utilisation en thérapie.

8. Composition selon l'une quelconque des revendications 1 à 6, destinée à une utilisation dans le traitement d'un cancer, ou destinée une utilisation dans l'administration d'une molécule de médicament à un site tumoral.

9. Composition destinée à une utilisation selon la revendication 8, dans laquelle le cancer est un cancer du cerveau, un cancer ovarien ou un cancer du côlon.

10. Composition destinée à une utilisation selon la revendication 8, dans laquelle le cancer est un cancer résistant à la doxorubicine.

11. Nanoconjugué, comprenant :
a. une construction de micelle ;
b. un anticorps à chaîne unique à fragment variable (scFv) ;
c. un inhibiteur de NF-kb, qui est la curcumine et/ou un sel de celle-ci ; et
d. un inhibiteur de topoisomérase II, qui est la doxorubicine et/ou un sel de celle-ci ;
dans lequel la construction de micelle, l'anticorps à chaîne unique à fragment variable (scFv), l'inhibiteur de NF-kb et l'inhibiteur de topoisomérase II sont conjugués les uns aux autres.

12. Nanoconjugué de la revendication 11, dans lequel le nanoconjugué a une taille moléculaire comprise entre 20 nm et 50 nm.

13. Nanoconjugué de la revendication 11, dans lequel le nanoconjugué a une taille moléculaire comprise entre 10 nm et 20 nm.

14. Nanoconjugué de la revendication 11, dans lequel le nanoconjugué a une taille moléculaire inférieure à 20 nm.

15. Nanoconjugué de la revendication 11, dans lequel le nanoconjugué est entouré par une micelle.
